(19) Europäisches Patentamt
European Patent Office
Office européen des brevets

(11) **EP 4 361 135 A1**

(12) **EUROPEAN PATENT APPLICATION**
published in accordance with Art. 153(4) EPC

(43) Date of publication:
**01.05.2024 Bulletin 2024/18**

(21) Application number: **23834500.3**

(22) Date of filing: **11.05.2023**

(51) International Patent Classification (IPC):
***C07D 311/72*** *(2006.01)* ***C07C 403/20*** *(2006.01)*
***C07D 207/16*** *(2006.01)* ***A61P 39/06*** *(2006.01)*
***C07C 391/02*** *(2006.01)* ***B01J 31/02*** *(2006.01)*
***A61K 8/67*** *(2006.01)* ***A61Q 19/08*** *(2006.01)*

(52) Cooperative Patent Classification (CPC):
**A61K 8/67; A61P 39/06; A61Q 19/08; B01J 31/02; C07C 391/02; C07C 403/20; C07D 207/16; C07D 311/72;** Y02P 20/55

(86) International application number:
**PCT/CN2023/093505**

(87) International publication number:
**WO 2024/007726 (11.01.2024 Gazette 2024/02)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.11.2022 CN 202211480897**

(71) Applicant: **Shanghai Coachchem Technology Co., Ltd**
**Shanghai 201500 (CN)**

(72) Inventors:
- **WU, Jiang**
  **Shanghai 201500 (CN)**
- **ZHANG, Wei**
  **Shanghai 201500 (CN)**
- **HAN, Tengfei**
  **Shanghai 201500 (CN)**
- **ZHOU, Baoping**
  **Shanghai 201500 (CN)**

(74) Representative: **Wang, Bo**
**Panovision IP**
**Ebersberger Straße 3**
**85570 Markt Schwaben (DE)**

(54) **SMALL-MOLECULE COMPOUND, AND USE THEREOF AND PREPARATION METHOD THEREFOR**

(57) The present invention relates to a small molecular compound and its application as an active ingredient in cosmetics, as well as its preparation method. The small molecular compound is represented by the following chemical formula:

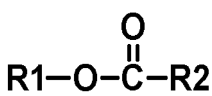

;Wherein, R1 is selected from compounds represented by the following formula:

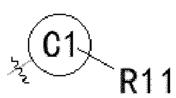

, C1 represents a five- or six-membered carbon ring or heterocycle, while R11 stands for alkyl or ester groups with multiple substitutions on the ring.

EP 4 361 135 A1

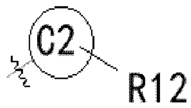

,

C2 represents an aromatic ring, while R12 stands for alkyl, ester, carbonyl, ether groups, or cyclic substituents formed by bridging two adjacent substituents on the ring with multiple substitutions.

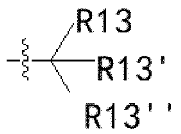

;

R13, R13', R13' , identical or different, are selected from hydrogen, ester groups, alkyl, alkenyl, and cycloalkenyl. R2 is chosen from

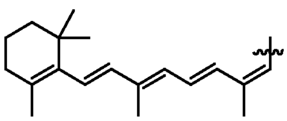 or 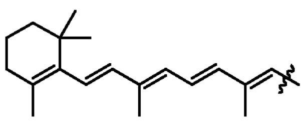.

This compound exhibits antioxidant properties when used as a reagent.

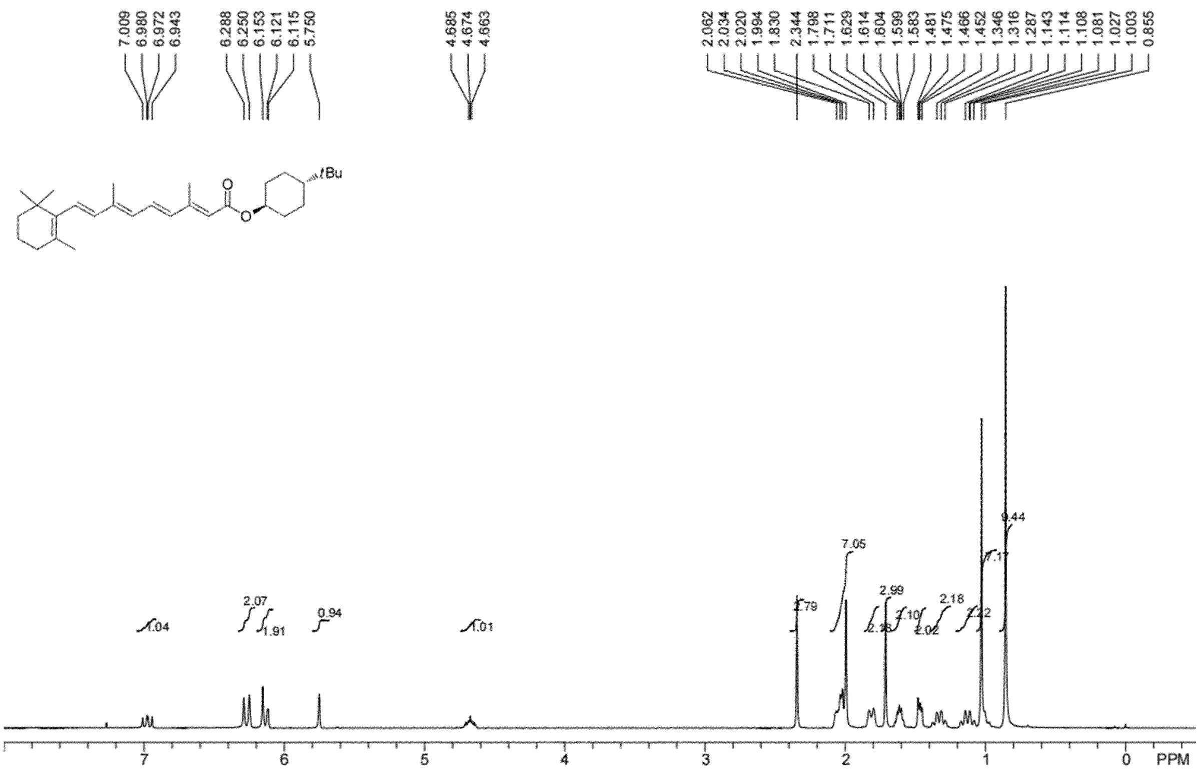


Figure 2-1

## Description

### Technical field:

The present invention pertains to cosmetic ingredients and the organic synthesis field, specifically involving a cosmetic active ingredient, its preparation method, and cosmetic applications.)

### Background technology:

With the upgrading of consumer demand, efficacy-based skincare has become one of the mainstream trends in cosmetics market. Traditional formulation techniques and the application of effective ingredients in cosmetics are increasingly challenged by new technologies, and the development and application of new materials pose one of the major challenges.

Currently, the majority of active ingredients in cosmetics on the market belong to single active molecules, meaning that the molecule of the active substance contains only one predominant organic structure and functional group. This structure exerts only one primary function on the skin. For example, tocopherol (Vitamin E) is a fat-soluble vitamin that directly scavenges free radicals in lipid-phase media, thereby interrupting the spread and diffusion of peroxidation of unsaturated fatty acids within the membrane’s lipid bilayer structure. It maintains the stability of unsaturated fatty acid composition on the membrane, effectively terminates the chain reaction of free radical-induced lipid peroxidation, protects cells from damage, and efficiently prevents oxidative aging of the skin.

On the other hand, retinoic acid belongs to the retinoid family, with the capability to regulate epidermal and stratum corneum metabolism. Its benefits have been proven for multiple indications, sparking immense public interest and demand for natural or synthetic retinoids.

However, traditional retinoid compounds can activate TRPV1 receptors, upregulate aquaporin 3 expression, disrupt cell junctions, and affect the expression of barrier-related proteins, leading to skin irritation such as peeling, erythema, itching, stinging, and burning sensation. Additionally, retinoid compounds often face instability issues, prone to decomposition or isomerization under light, temperature, oxygen exposure, making it difficult to maintain purity and consistency in content. Altogether, these characteristics pose significant difficulty and challenges in the application of retinoid products.

### Invention Summary:

The aim of this invention is to overcome the aforementioned shortcomings by providing a stable cosmetic active ingredient, along with its preparation method and cosmetic applications.

The present invention provides a small molecule compound characterized by the following chemical formula:

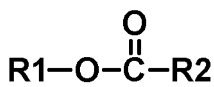
,

Wherein, R1 is selected from compounds represented by the following formula:

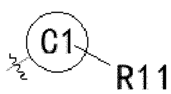
,

C1 is a five- or six-membered carbon ring or heterocycle, and R11 is any alkyl or ester group with multiple substitutions on the ring;

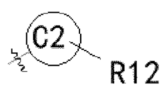
,

C2 is an aromatic ring, and R12 represents alkyl, ester, carbonyl, ether groups, or cyclic substituents formed by bridging two adjacent substituents on the ring with multiple substitutions;

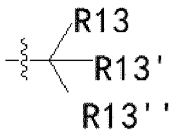,

R13, R13’, R13’ , identical or different, are selected from hydrogen, ester groups, alkyl, alkenyl, and cycloalkenyl;

R2 is chosen from

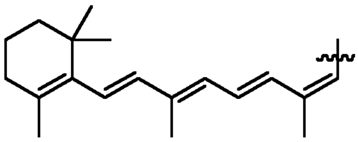 or 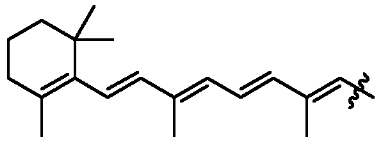.

The aforementioned alkyl is selected from chiral or achiral alkyl groups, preferably short-chain groups with a carbon atom count of up to 10. However, in the case of R12, bridged structures can also be formed based on long-chain alkyls with more than 10 carbon atoms.

The aforementioned ester groups are selected from short-chain groups with a carbon atom count of up to 10, adopting the formula -C(O)-O-R or -O-C(O)-R.

The mentioned ether groups are derived from short-chain groups containing a maximum of 10 carbon atoms, presented as -O-R.

The aforementioned alkene groups are selected from short-chain groups comprising up to 10 carbon atoms.

The aforementioned cycloalkenyl groups are selected from tetra-, penta-, or hexa-membered cycloalkenyl rings.

Moreover, the small molecule compound provided by this invention is further characterized by: )

R1 is chosen from

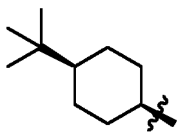

or

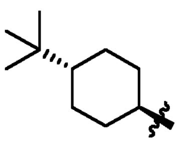 or 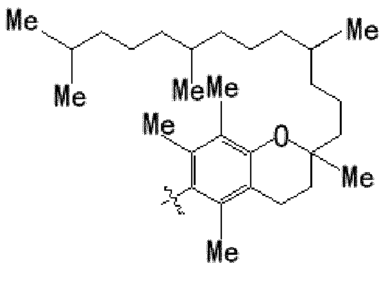 or 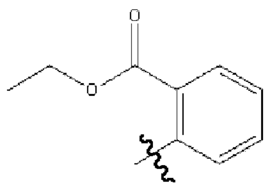 or 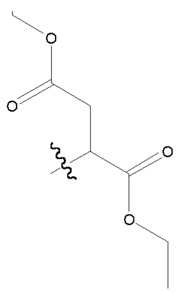

or

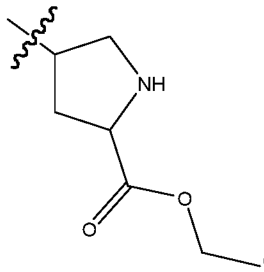 or 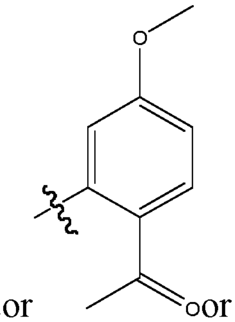 or 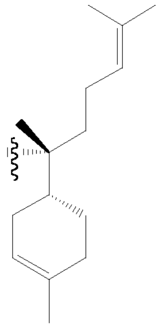 .

Furthermore, the small molecule compound provided by the present invention is further characterized by: The aforementioned small molecule compound is selected from one or several compounds represented by the following structures:

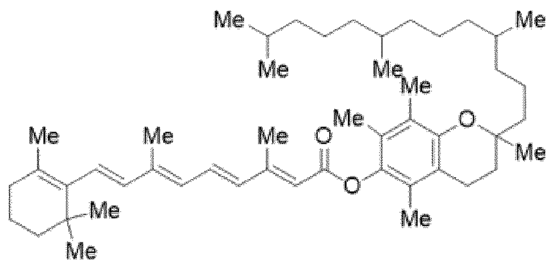

compound 1;

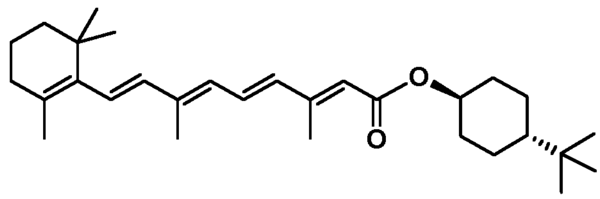

compound 2;

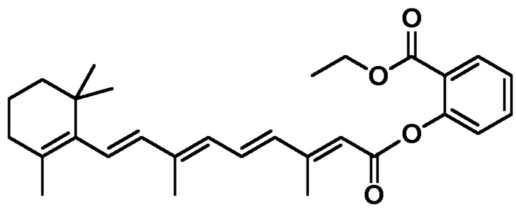

compound 3;

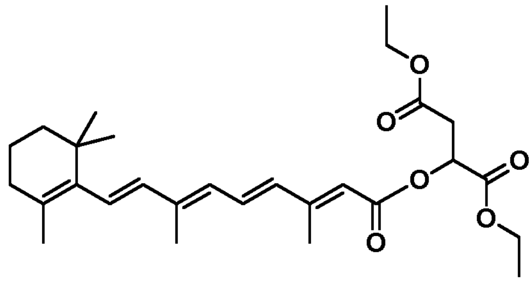

compound 4;

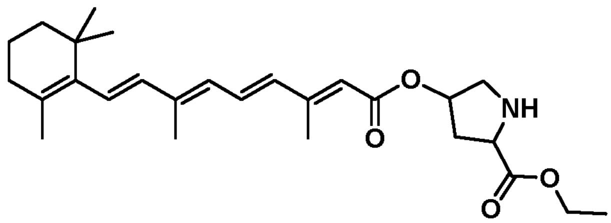

compound 5;

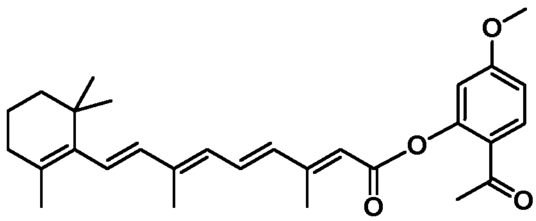

compound 6;

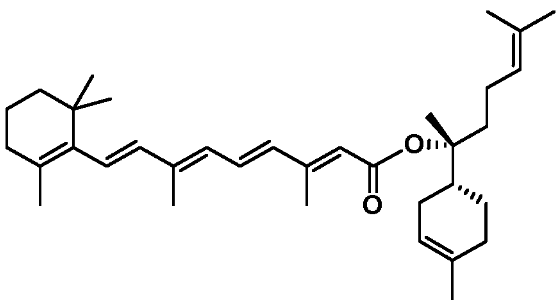

compound 7;

Furthermore, the small molecule compound provided by the present invention is further characterized by: The aforementioned small molecule compound serves as an active ingredient utilized in cosmetics.

Furthermore, the small molecule compound provided by the present invention is further characterized by containing at least one of the following uses:

A. Serving as an antioxidant.
B. Serving as an antioxidant for use in cosmetics.

Furthermore, the small molecule compound provided by the present invention is further characterized by.

A. Serving as a DPPH radical scavenger.
B. Serving as a DPPH radical scavenger for use in cosmetics.

Furthermore, the small molecule compound provided by the present invention is further characterized by.

A. Serving as an inhibitor of intracellular reactive oxygen species (ROS)
B. Serving as an inhibitor of intracellular reactive oxygen species (ROS) for use in cosmetics

Furthermore, the preparation method of the small molecule compound provided by the present invention is described as follows:
Using a selenium-containing catalyst, under its catalytic action, carboxylic acids containing active unit A and alcohols containing active unit B react to form small molecular compounds containing both active units A and B simultaneously.
Wherein, the carboxylic acid containing active unit A mentioned above has the general formula R2COOH.
The alcohol containing active unit B mentioned above has the general formula R1OH.
Furthermore, the preparation method of the small molecule compound provided by the present invention is described as follows:
The structural formula of the selenium-containing catalyst is as follows:

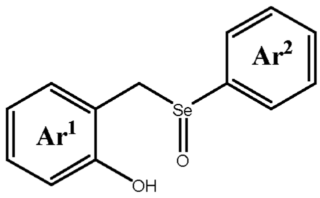 ;

Wherein, the

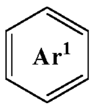 and 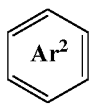

are benzene ring that may or may not contain substituents; these substituents are chosen from alkyl, alkoxy, fluoroalkyl, halogens, cyano, and amino groups.

### The function and effect of this invention

In existing research, it has been found that the isolated use of retinoid compounds demonstrates excellent antioxidant efficacy, improving skin elasticity and firmness when applied to the skin's epidermis. However, retinoids can activate TRPV1 receptors, leading to adverse effects on the skin such as peeling, erythema, itching, stinging, and a burning sensation, limiting their suitability for a broader user base and requiring tolerance development. Additionally, their poor stability imposes significant challenges on formulation development and design, considerably restricting their application scope.
In the research conducted for this invention, it has been discovered that although retinoic acid (vitamin A acid) exhibits strong skin irritability, effective alleviation of this irritability is achievable through side-chain esterification modification. For instance, derivatives such as retinyl esters modified with 4-tert-butylcyclohexanol significantly mitigate the irritative effects observed with the individual use of retinoic acid. Moreover, the esterified retinoic acid displays enhanced stability. It exhibits excellent stability in harsh and accelerated environments, facilitating its convenient incorporation into formulations or products. This improved stability further facilitates commercial storage and longevity.
Specifically, in the research of this invention, it was found that 4-tert-butylcyclohexanol, a derivative of menthol, selectively antagonizes TRPV1 receptors, inhibiting their expression, activation, and effects. Consequently, it modulates skin sensation, reduces vascular hyperreactivity, alleviates skin inflammation responses, and mitigates symptoms of skin sensitivity, thereby potentially improving the irritative effects caused by retinoids.
However, the efficacy of 4-tert-butylcyclohexanol as an antagonist of the TRPV1 receptor is also quite limited when applied alone. While formulation design may involve the concurrent use of both retinoids and 4-tert-butylcyclohexanol to ameliorate the irritative effects induced by retinoids, the stability of retinoids remains considerably challenging, posing difficulties in preserving their activity.

Therefore, in the design of this invention, to address the aforementioned issues, consideration has been given to the integration of the efficacious functional groups of both 4-tert-butylcyclohexanol and retinoids through specific conjugation techniques. This integration results in a more stable, less irritative active molecule, eliminating the necessity for tolerance development, and facilitating its application. Consequently, it broadens the scope of its application and holds promising prospects.

Based on the aforementioned design concept, this invention offers a cosmetic active ingredient featuring characteristic groups derived from two active molecules, exerting antioxidant effects. This compound holds promise as a new cosmetic efficacy material and exhibits excellent prospects for application.

As a result, esterified retinoic acid, akin to 4-tert-butylcyclohexanol retinyl ester, displays promising prospects in the cosmetics field. Further research into an efficient method that conserves energy, diminishes safety concerns, minimizes toxicity risks in the production process, and enhances the yield of 4-tert-butylcyclohexanol retinyl ester is pivotal. Not only will this aid in delving deeper into its functionalities and applications, but it also holds substantial economic value and market potential.

This invention achieves the coupling of two cosmetic active molecules using an organic selenium catalyst. The reaction conditions are mild and do not require strong acids or dehydrating agents, necessitating only a catalytic amount of organic selenium. This method is cost-effective, technologically simple, environmentally friendly, and suitable for large-scale production.)

**Figure Description**

Figure 1-1: Schematic of the Reaction Process;
Figure 1-2: Mechanism Diagram;
Figure 2-1: Proton NMR of Compound Corresponding to Example 2-1;
Figure 2-2: Carbon NMR of Compound Corresponding to Example 2-1;
Figure 2-3: Elemental Analysis of Compound Corresponding to Example 2-1;
Figure 2-4: Mass Spectrum of Compound Corresponding to Example 2-1;
Figure 3: Reference Curve for Ascorbic Acid (Vitamin C) DPPH Free Radical Scavenging Activity;
Figure 4: Antioxidant Activity of Retinyl-*trans*-4-tert-butylcyclohexanol Ester against DPPH Free Radicals ***: $p < 0.001$ compared to the control group;
Figure 5: Elimination of Intracellular Reactive Oxygen Species (ROS) by Retinyl-*trans*-4-tert-butylcyclohexanol Ester ###: $p < 0.001$ compared to the Control group; ***: $p < 0.001$ compared to the UVB control group; NS: indicates no statistical difference, $p \geq 0.05$
Figure 6: Skin Responses of 30 Subjects under Different Observational Conditions;
Figure 7: Stability Comparison of Retinyl-*trans*-4-tert-butylcyclohexanol Ester and Retinol under Light Conditions;
Figure 8: Stability Comparison of Retinyl-*trans*-4-tert-butylcyclohexanol Ester and Retinol at 50°C;
Figure 9: Stability Comparison of Retinyl-*trans*-4-tert-butylcyclohexanol Ester and Retinol at 45°C;
Figure 10: Stability Comparison of Retinyl-*trans*-4-tert-butylcyclohexanol Ester and Retinol at Room Temperature;
Figure 11: Stability Comparison of Retinyl-*trans*-4-tert-butylcyclohexanol Ester and Retinol at 4°C.

**Specific Implementation Methods:**

For the preparation method of the aforementioned small molecule compound, traditional esterification methods can be employed. However, their yield and purity might not be optimal, particularly when the compound possesses chirality. Therefore, in this specific case, a novel preparation method is proposed. This method involves the use of a selenium-containing catalyst, where, under its catalytic action, a carboxylic acid containing active unit A reacts with an alcohol containing active unit B, resulting in the formation of a small molecule compound containing both active units A and B simultaneously.

The carboxylic acid containing the aforementioned active unit A has the general formula R2COOH.

The alcohol containing the aforementioned active unit B has the general formula RICH.

The reaction process is illustrated in Figure 1-1.

During the selenium-catalyzed reaction process, the carboxylic acid containing active unit A and the alcohol containing active unit B, under the catalytic influence of a selenium-containing catalyst, undergo reflux reaction at 60-90°C for 0.5-10 hours to obtain the desired product.

The molar ratio between the carboxylic acid containing active unit A and the alcohol containing active unit B is typically 1:1.

The catalyst is generally used in an amount of 1-10% based on the molar quantity of the carboxylic acid

containing active unit A. The reaction is usually carried out in a solvent with a boiling point ranging from 60-90°C.

The structural formula of the aforementioned selenium-containing catalyst is as follows:

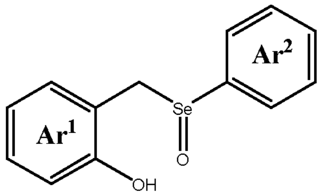;

Among which, the above

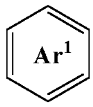 and 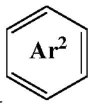

are a benzene ring with or without substituents.

The above substituents are selected from alkyl, alkoxy, fluoroalkyl, halogens, cyano, and amino.

The above selenium-containing catalyst is preferably selected from at least one of the catalysts represented by catalyst 1 or catalyst 2

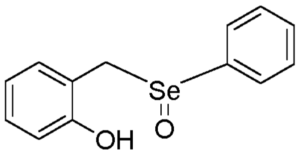

Catlyst 1

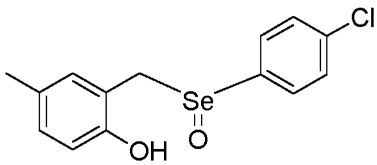

Catlyst 2 .

The specific reaction mechanism is illustrated in Figure 1-2.

The aforementioned selenium-containing catalyst is obtained from the reaction between ortho-hydroxybenzyl halide and diphenyldiselenide. The structure of the aforementioned ortho-hydroxybenzyl halide is as follows:

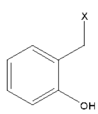;

The structure of the aforementioned diphenyldiselenide is as follows

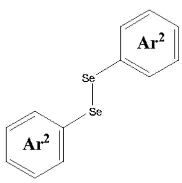.

X is preferably Br. The specific reaction equation is as follows:

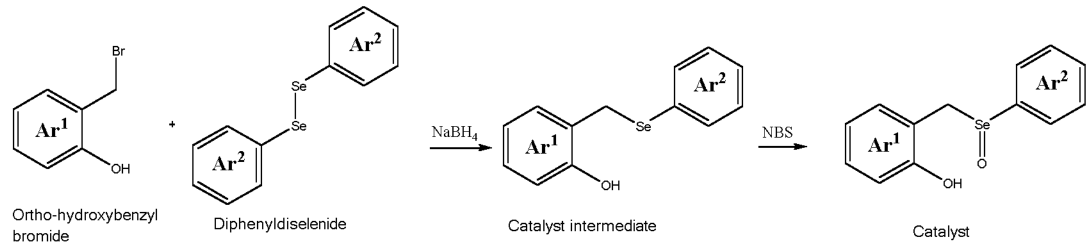


Ortho-hydroxybenzyl bromide Diphenyldiselenide Catalyst intermediate Catalyst

The preparation method of the aforementioned selenium-containing catalyst is as follows:

S1. At room temperature, add a reducing agent to diphenyldiselenide and stir until it clarifies.
S2. At room temperature, add ortho-hydroxybenzyl halide to the reaction solution from S1. After reacting for 10-36 hours, the resulting solution undergoes acidification, extraction, drying, filtration, and concentration to obtain the catalyst intermediate.
S3. Dissolve the catalyst intermediate obtained in S2, cool it to below 0 degrees Celsius, add NBS, and react for 1-10 hours. Then, quench the reaction with an alkali solution. Extract, dry, filter, concentrate, and recrystallize to obtain the target product.

Further, the method for preparing a small molecule compound provided by the present invention also comprises the following features:
The molar ratio of the aforementioned ortho-hydroxybenzyl halide to the diaryl diselenide ranges from 1:1 to 1:2.
The molar ratio of the aforementioned ortho-hydroxybenzyl halide to the reducing agent is between 1:2 to 1:3.
The molar ratio of the aforementioned ortho-hydroxybenzyl halide to N-bromosuccinimide (NBS) is between 1:2 to 1:5.
Based on the above scheme, specific experiments are conducted as shown below:)

Example 1. General synthetic steps of the organic selenium catalyst:

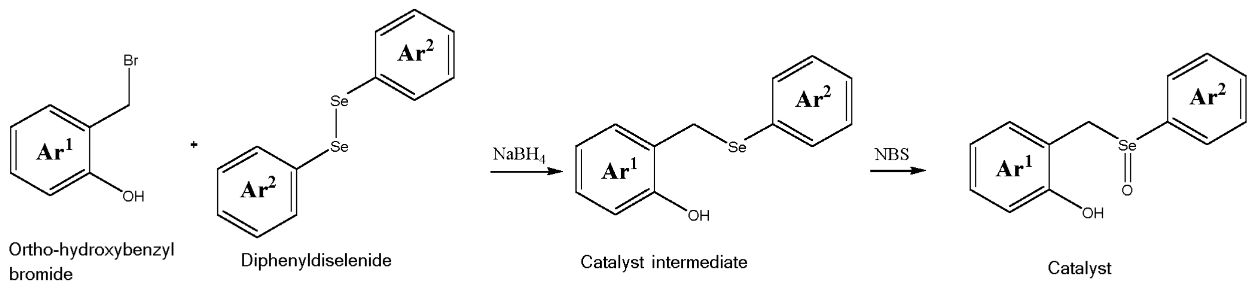


Ortho-hydroxybenzyl bromide Diphenyldiselenide Catalyst intermediate Catalyst

At room temperature, to a solution of 120 mmol of diaryl diselenide in 200 mL of tetrahydrofuran (THF), sodium borohydride (200 mmol) was added dropwise. The reaction continued until the solution became clear. Then, 100 mmol of 2-bromobenzyl alcohol was added, and the reaction was allowed to proceed for an additional 36 hours. Afterward, the reaction mixture was acidified with hydrochloric acid, extracted, dried, filtered, concentrated to yield the catalyst intermediate.
Subsequently, the obtained catalyst intermediate was dissolved in a mixture of methanol and dichloromethane. The solution was cooled to below 0 degrees Celsius, and N-bromosuccinimide (NBS) (200 mmol) was added. After a reaction time of 1 hour, the reaction was quenched using a 10% aqueous solution of sodium hydroxide. The resulting mixture was then extracted with dichloromethane, dried, filtered, and concentrated to obtain the crude product. Finally, the crude product was recrystallized from a mixture of n-hexane and dichloromethane to obtain the target product.)

Example 1-1. Preparation of Catalyst 1

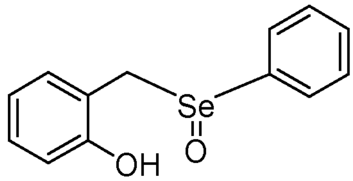

catlyst1

According to the general synthesis procedure for the organic selenium catalyst, diphenyldiselenide and benzyl bromide without any other substituents were selected. This yielded Catalyst 1 with a purity of 98.5% and a yield of 83%.

Characterization of Compounds: $^1$H NMR (400 MHz, $CDCl_3$) $\delta$ = 9.71 (s, 1H), 7.32 - 7.36 (m, 5H), 6.97-7.03 (m, 2H), 6.73-6.83 (m, 2H), 2.65 (s, 2H). $^{13}$C NMR (400 MHz, $CDCl_3$) $\delta$ = 157.3, 135.1, 132.3, 131.1, 130.9, 127.5, 125.5, 124.7, 121.0, 116.2, 62.7. m/z = 280.1. Elemental analysis: C, 56.01; H, 4.39.

Example 1-2. Preparation of Catalyst 2:

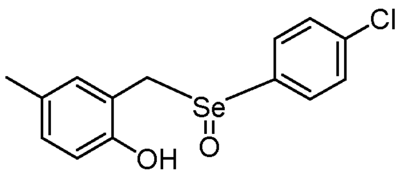

catalyst2

Following the general synthesis steps for the organic selenium catalyst, diphenyldiselenide was chosen as the diselenide compound, and 2-bromo-4-methylphenol was used as the 4-hydroxybenzyl bromide. This procedure resulted in the formation of catalyst 2, with a yield of 81% and a purity of 99%.

Characterization of Compounds: $^1$H NMR (400 MHz, $CDCl_3$) $\delta$ = 9.64 (s, 1H), 7.44 (d, J = 7.5 Hz, 2H), 7.25 (d, J = 7.5 Hz, 2H), 6.89-6.91 (m, 2H), 6.75 (d, J = 7.5 Hz, 1H), 2.63 (s, 2H), 2.34 (s, 3H). $^{13}$C NMR (400 MHz, $CDCl_3$) $\delta$ = 154.2, 134.6, 133.1, 132.0, 130.7, 128.9, 127.5, 124.5, 116.1, 63.5, 21.8. m/z = 328.9. Elemental analysis : C, 51.43; H, 4.21.

Example 2.

Example 2-1. Synthesis of trans-4-tert-butylcyclohexanol and retinoic acid:

In a reaction flask, sequentially add 200 mL of toluene, retinoic acid (100 mol), trans-4-tert-butylcyclohexanol (100 mol), and organic selenium catalyst (5 mol). Reflux the mixture at elevated temperature for 18 hours, then extraction three times using water and ethyl acetate. Remove the aqueous layer and dry the organic layer with anhydrous sodium sulfate. Evaporate the solvent using a rotary evaporator, followed by purification through silica gel column chromatography to obtain the pure spliced product:

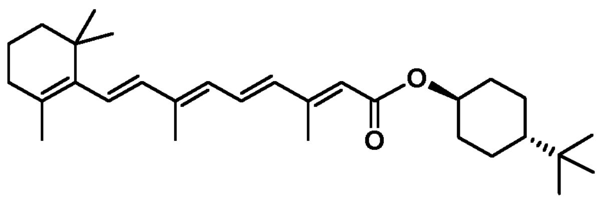

According to the actual differences in reactants, the molar ratio between trans-4-tert-butylcyclohexanol and retinoic acid can be adjusted in the range of 1-2:1. The molar amount of the organic selenium catalyst is 2-20% based

on the hydroxyl group-containing active compound. As a preferred embodiment: In this particular embodiment, the compound with the following structure was chosen as the catalyst:

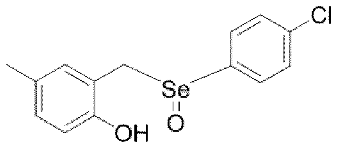

The above catalyst was synthesized according to the synthetic steps of the organic selenium catalyst in Example 1-2, and after synthesis, it exhibited a nuclear magnetic resonance (NMR) purity of 99%. Then, the aforementioned catalyst was utilized in a specific catalytic reaction experiment. Following the general steps for splicing two active compounds, the organic selenium catalyst chosen was the aforementioned catalyst 1, resulting in the production of retinoic acid *trans*-4-tert-butylcyclohexyl ester with a yield of 85% and a purity of 99.2%.

Characterization of Compounds Cfigure2-1, 2-4): $^1$H NMR (400 MHz, $CDCl_3$) $\delta$ = 7.01-6.94 (m, 1H), 6.29-6.11 (m, 4H), 5.75 (s, 1H), 4.68-4.66 (m, 1H), 2.34 (s, 3H), 2.06-1.83 (m, 4H), 1.99 (s, 3H), 1.71 (s, 3H), 1.80-1.58 (m, 6H), 1.48-0.81 (m, 5H), 1.00 (s, 6H), 0.86 (s, 9H).

$^{13}$C NMR (400 MHz, $CDCl_3$) $\delta$ = 166.8, 152.4, 139.4, 137.8, 137.4, 135.4, 130.8, 130.0, 129.7, 128.6, 119.3, 77.5, 77.2, 76.8, 73.0, 47.3, 39.7, 34.4, 33.2, 32.4, 29.1, 27.7, 25.6, 21.9, 19.3, 13.9, 13.0.
Elemental analysis: C,82.00; H,10.67.
m/z: 439.4

Example 2-2. Synthesis of the compound resulting from the splicing of retinoic acid and hydroquinone:

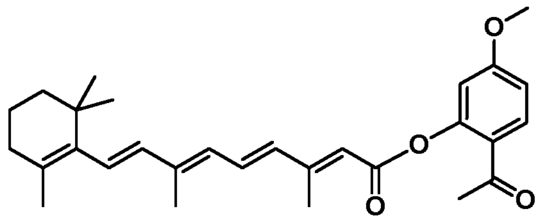

According to the general steps for splicing two active compounds, retinoic acid was chosen as the carboxylic acid active compound, hydroquinone was selected as the hydroxyl group active compound, and catalyst 1 was employed as the organic selenium catalyst. The target compound was obtained with a yield of 87% and a purity of 99.2%.)

Characterization of Compounds: $^1$H NMR (400 MHz, $CDCl_3$) $\delta$ = 7.73 (d, J = 8 Hz, 1H), 7.52 (s, 1H), 7.03-6.97 (m, 1H), 6.91 (d, J = 8 Hz, 1H), 6.33-6.15 (m, 4H), 5.89 (s, 1H), 3.87 (s, 3H), 2.67 (s, 3H), 2.34 (s, 3H), 2.08-1.80 (m, 6H), 1.98 (s, 3H), 1.72 (s, 3H), 1.00 (s, 6H).

$^{13}$C NMR (400 MHz, $CDCl_3$) $\delta$ = 197.1, 167.5, 166.8, 152.4, 150.4, 139.4, 137.8, 137.4, 135.4, 133.6, 130.8, 130.0, 129.7, 128.6, 119.3, 115.9, 112.3, 105.8, 73.0, 39.7, 34.4, 33.2, 32.4, 29.1, 21.9, 19.3, 13.9, 13.0.
Elemental analysis: C, 77.41; H, 8.01.
m/z: 449.2

Example 2-3. Synthesis of Compound from Retinoic Acid and Bisabolol

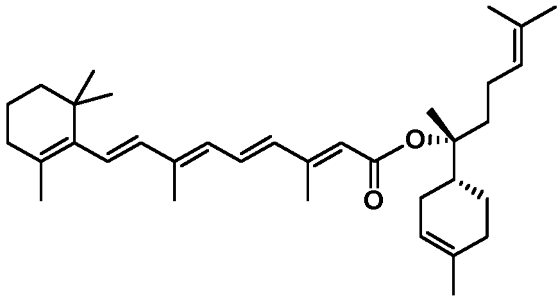

According to the general splicing steps for two active compounds, retinoic acid was used as the carboxylic acid active compound, and retinol was chosen as the hydroxyl active compound. Catalyst 1 was employed as the organic selenium catalyst. The target compound was obtained with a yield of 81% and a purity of 99.5%.
Characterization of Compounds: $^1$H NMR (400 MHz, $CDCl_3$) $\delta$ = 7.03-6.97 (m, 1H), 6.91 (d, J = 8 Hz, 1H), 6.33-6.15 (m, 4H), 5.89 (s, 1H), 5.33 (s, 1H), 5.02 (s, 1H), 2.34 (s, 3H), 2.12-1.83 (m, 13H), 1.98 (s, 3H), 1.82 (s, 3H), 1.72 (s, 3H), 1.68 (s, 3H), 1.64 (s, 3H), 1.61-1.33 (m, 4H), 1.43 (s, 3H), 1.00 (s, 6H).

$^{13}$C NMR (400 MHz, $CDCl_3$) $\delta$ =167.5, 166.8, 152.4, 139.4, 137.8, 137.4, 135.4, 134.2, 133.6, 131.3, 130.8, 130.0, 129.7, 128.6, 124.5, 121.4, 119.3, 74.7, 73.0, 42.6, 39.7, 34.4, 33.2, 32.4, 31.1, 29.1, 26.9, 24.3, 23.9, 22.4, 22.1, 21.9, 19.3, 13.9, 13.0.
Elemental analysis: C, 83.31; H, 10.45.
m/z: 505.8

Example 3. **DPPH Antioxidant Assay of Retinyl-*trans*-4-tert-butylcyclohexanol Ester:**

3.1 Experimental Principle: The DPPH radical scavenging assay is an in vitro method used to evaluate antioxidant activity. DPPH is a stable free radical in organic solvents and appears purple in methanol or ethanol, with maximum absorption at a wavelength of 517 nm. The DPPH assay is based on the ability of an antioxidant to donate an electron to the DPPH radical, converting its purple color to yellow. The degree of absorbance change at 517 nm corresponds to the extent of the free radical scavenging activity, meaning the stronger the scavenging ability of the antioxidant, the lower the absorbance.

**3.2 Experimental Materials Reagents:**

DPPH (Sigma), PBS (Gibco), anhydrous ethanol (National Pharmaceutical Reagent), petroleum ether (National Pharmaceutical Reagent), Vitamin C (CNW), anhydrous ethanol (National Pharmaceutical Reagent). Main Equipment: Microplate Reader (Tecan, Spark), Microplate Shaker (Kylin-Bell, TS-92).
3.3 In vitro DPPH Free Radical Scavenging Assay 3.3.1 Preparation of the DPPH Free Radical Scavenging Reference Curve Using ascorbic acid (VC) as a system reference, dilute it with PBS to obtain five concentrations: 12.5, 25, 50, 100, and 200 μg/mL. Perform the test and calculations according to the procedure outlined in 3.3.2. Plot the reference curve with the concentration of the reference substance on the x-axis and the DPPH free radical scavenging rate on the y-axis.
3.3.2 In vitro DPPH Free Radical Scavenging Test Prepare the samples into the respective concentrations of test solutions. Prepare the reaction system by adding the specified amount of each reagent mentioned in Table 1, mix thoroughly. For each concentration, set up three replicate wells and one blank control well.

Table 1. Reaction System for DPPH Free Radical Scavenging Test (units in μL)

| (μL) | C1 | C2 | T1 | T2 |
|---|---|---|---|---|
| DPPH Ethanol Solution | 180 | 0 | 180 | 0 |
| Test Sample | 0 | 0 | 20 | 20 |
| Anhydrous Ethanol | 0 | 180 | 0 | 180 |
| PBS | 20 | 20 | 0 | 0 |

The reaction system is placed at room temperature and allowed to react in the dark for 30 minutes. After the

reaction is complete, the absorbance (OD value) is measured at 515nm. The formula to calculate the scavenging rate of the sample against DPPH free radicals is as follows:

Sample's DPPH Free Radical Scaveging Rate = [(C1-C2) - (T1-T2)]/(C1- C2) × 100%

Where:

C1 = Absorbance value of the blank with DPPH
C2 = Absorbance value of the blank without DPPH
T 1 = Absorbance value of the sample with DPPH
T2 = Absorbance value of the sample without DPPH

**3.4 In vitro DPPH radical scavenging assay result**

3.4.1 In Vitro DPPH Free Radical Scavenging Test Results The system reference curve for DPPH free radical scavenging is presented in Table 1 and Figure 3.

Table 2 System Reference Results Analysis

| Concentration (μg/mL) | 200 | 100 | 50 | 25 | 12.5 |
|---|---|---|---|---|---|
| (%)DPPH Free Radical Scavenging Rate (%) | 101.28 | 101.32 | 64.58 | 30.26 | 20.55 |
| | 92.01 | 95.11 | 65.09 | 31.17 | 18.55 |
| | 108.75 | 97.07 | 62.73 | 32.98 | 20.32 |

3.4.2 Results of In Vitro DPPH Free Radical Scavenging Test Refer to Table 3 and Figure 4

Table 3: Analysis of DPPH Free Radical Scavenging Test Results

| **Sample** | **Sample Concentration (% v/v)** | **DPPH Free Radical Scavenging Rate (% v/v)** | ***p*** | **Signific ance** |
|---|---|---|---|---|
| Retinyl 4-*trans*-t-Butyl cyclohexanol | 0 | 0.00±1.22 | / | / |
| | 4 | 83.96±1.22 | <0.001 | *** |
| | 2 | 48.12±1.07 | <0.001 | *** |
| | 0.5 | 36.72±1.98 | <0.001 | *** |
| | 0.1 | 15.91±1.25 | <0.001 | *** |
| | 0.02 | 9.87±3.44 | <0.001 | *** |
| Note: Data presented as mean ± SD. When conducting statistical analysis using the t-test, significance is denoted by *, where p < 0.001 is represented as *** when comparing different concentrations of Retinyl 4-trans-t-Butylcyclohexanol experimental groups with the blank control group. | | | | |

Conclusion: Retinyl 4-*trans*-t-Butylcyclohexanol at concentrations ranging from 0.02% to 4% demonstrates a statistically significant increase in DPPH free radical scavenging compared to the control group ($p < 0.001$), indicating its antioxidative capacity.

**Example 4 Implementation of ROS scavenging test of Retinyl 4-*trans*-t-Butylcyclohexanol:**

4.1 Experimental Objective

Oxidation represents the greatest threat to skin aging, primarily caused by environmental stressors such as UV radiation, pollution, smoke, and daily stress. Among these factors, UV radiation stands as the most significant contributor. Skin exposed to UV radiation generates an excess of reactive oxygen species (ROS) within cells, triggering

the expression of genes related to aging, instigating an inflammatory cascade, and reducing the expression of elastin and collagen proteins, leading to manifestations of photoaging like skin laxity and wrinkles. Photoaging predominantly occurs in the dermis. Therefore, this test utilizes fibroblast cells as a test system to evaluate changes in ROS levels and assess whether Retinyl 4-t-Butylcyclohexanol test samples possess antioxidant effects in reducing ROS levels.

4.2 Experimental Design

UVB irradiation is administered to immortalized human keratinocytes (HaCat cells) to induce intracellular reactive oxygen species (ROS) generation. The UVB dose applied to HaCat cells is 20 mJ/cm2. Following the UVB treatment, varying concentrations of Retinyl 4-t-Butylcyclohexanol test samples are added to the cells and incubated for 24 hours. ROS levels within the cells are assessed using a ROS detection kit.

**4.3 Experimental Results**

Graph Pad Prism was used for statistical plotting, and the results were presented as Mean±SD. Statistical analysis was conducted using the t-test. A significance level of $p<0.05$ was considered statistically significant, where *p<0.05, **p<0.01 (0.005<**p<0.01), and ***p<0.001, indicating increased significance as the p-value decreases. When employing the t-test for statistical analysis, the comparison between the UVB control group and the Control group was represented as # (p<0.001 indicated as ###). For the experimental groups with varying concentrations of Retinyl-*trans*-4-tert-Butylcyclohexanol compared to the UVB control group, significance was represented by * ("ns" indicated no statistical difference, p≥ 0.05; p<0.001 indicated as ***).

The results of the Retinyl-*trans*-4-tert-Butylcyclohexanol intracellular reactive oxygen species (ROS) scavenging experiment are illustrated in Figure 5 below. Conclusion: UVB significantly increased the ROS level in Hacat cells. Retinyl-*trans*-4-tert-Butylcyclohexanol effectively reduced the UVB-induced intracellular ROS levels compared to the control group, showing statistical significance (p<0.001), thereby demonstrating antioxidative capabilities.

Example 5. **Retinyl-trans-4-tert-Butylcyclohexanol Patch Test:**

5.1 Materials and Methods

5.1.1 Test substance: 2% Retinyl-*trans*-4-tert-Butylcyclohexanol oil solution (the carrier oil here is Caprylic/Capric Triglyceride).

5.1.2 Negative control: Filter patch.

5.1.3 Subjects: A total of 30 individuals, 15 males and 15 females, aged between 22 to 55 years old, with an average age of 40.73±1.76 years old, meeting the selection criteria for volunteers. (To prevent the possibility of subjects dropping out during the study, two alternative candidates, one male and one female, were selected for this experiment.

5.1.4 Patch test method: Qualified patch test equipment was used, employing a closed patch test method. Approximately 0.020mL to 0.025mL (liquid) of the test substance was placed onto the patch test apparatus and applied on the subjects' backs using hypoallergenic adhesive tape. After 24 hours, the test substance was removed, and skin reactions were observed at 0.5, 24, and 48 hours post-removal. Skin reactions were recorded according to the current effective standards for grading skin reactions in accordance with established technical specifications.

**5.2 Test Results**

The patch test results on human skin showed negative reactions for all 30 subjects. The summarized results are presented in Table 4.

Table 4 Summary of Human Skin Patch Test Results for Cosmetics

| Groups | Number of Subjects | Observation Time | Number of Subjects with Different Skin Reactions in Patch Test | | | | |
|---|---|---|---|---|---|---|---|
| | | | 0 | 1 | 2 | 3 | 4 |
| Test Substance | 30 | 0.5 | 30 | 0 | 0 | 0 | 0 |
| | | 24 | 30 | 0 | 0 | 0 | 0 |
| | | 48 | 30 | 0 | 0 | 0 | 0 |

(continued)

| Groups | Number of Subjects | Observation Time | Number of Subjects with Different Skin Reactions in Patch Test | | | | |
|---|---|---|---|---|---|---|---|
| | | | 0 | 1 | 2 | 3 | 4 |
| Control | 30 | 0.5 | 30 | 0 | 0 | 0 | 0 |
| | | 24 | 30 | 0 | 0 | 0 | 0 |
| | | 48 | 30 | 0 | 0 | 0 | 0 |
| Note: Skin reaction observations for the first 30 subjects at different observation times are depicted in Figure 6. | | | | | | | |

6. Stability Comparison between Retinyl-*trans*-4-tert-Butylcyclohexanol and Retinol

6.1 Experimental Purpose

To compare the stability of Retinyl-*trans*-4-tert-Butylcyclohexanol and retinol (selected as a representative of retinol) under different conditions.

6.2 Experimental Design

A certain amount of retinol and Retinyl-*trans*-4-tert-Butylcyclohexanol is taken and placed under conditions of light exposure, 4°C, 25°C, 45°C, and 50°C for 28 days. Samples are periodically collected to determine their content and examine the influence of light and temperature on their stability.

6.3 Instruments and Equipment

High-Performance Liquid Chromatography with Diode Array Detection (HPLC-DAD), Electronic Balance (precision of 0.01 mg), Chromatographic Column (Welch Ultimate series XB-C18 column, 250mm*4.6mm, 5μm), Several volumetric flasks.

6.4 Reagents, Solutions, and Control Samples

Reagents: Acetonitrile (chromatographic grade), Formic acid (chromatographic grade), Ultrapure water.
Control Sample 1: Retinol, purity above 99.5%, Manufacturer: Self-made by Coachchem Laboratory (-20°C, light-resistant and vacuum-sealed).
Control Sample 2: Retinyl-*trans*-4-tert-Butylcyclohexanol, purity above 99.0%, Manufacturer: Self-made by Kechin Laboratory (-20°C, light-resistant and vacuum-sealed).
Blank Solvent: Acetonitrile - 0.1% Formic acid water solution (95/5).

6.5 Sample Preparation

6.5.1 Control Sample 1 Solution: Accurately weigh approximately 10.0 mg of retinol crystal pure substance control sample into a 20mL brown volumetric flask. Add antioxidant BHT (butylated hydroxytoluene) accordingly, then add an appropriate amount of acetonitrile, ultrasonicate while avoiding light, dilute to the mark, shake well, and prepare two parallel samples.
6.5.2 Control Sample 2 Solution: Accurately weigh approximately 10.0 mg of Retinyl-*trans*-4-tert-Butylcyclohexanol crystal pure substance control sample into a 20mL brown volumetric flask. Add antioxidant BHT accordingly, then add an appropriate amount of acetonitrile, ultrasonicate while avoiding light, dilute to the mark, shake well, and prepare two parallel samples.
6.5.3 Retinyl-*trans*-4-tert-Butylcyclohexanol Sample Solution: Accurately weigh approximately 0.1g of Retinyl-*trans*-4-tert-Butylcyclohexanol samples obtained under conditions of light exposure, 4°C, 25°C, 45°C, and 50°C into a 100mL brown volumetric flask. Add an appropriate amount of acetonitrile while avoiding light, ultrasonicate, dilute to the mark, shake well, and prepare two parallel samples for each condition.
6.5.4 Retinol Sample Solution: Accurately weigh approximately 0.1g of retinol samples obtained under conditions of light exposure, 4°C, 25°C, 45°C, and 50°C into a 100mL brown volumetric flask. Add an appropriate amount of acetonitrile while avoiding light, ultrasonicate, dilute to the mark, shake well, and prepare two parallel samples for each condition.

6.6 Chromatographic Conditions

6.6.1 Retinol Chromatographic Conditions Mobile Phase:

Phase A of acetonitrile; phase B of 0.1% formic acid-water.
Column Temperature: 25°C
Flow Rate: 1.0mL/min
Wavelength: 325nm
Injection Volume: 10μL Gradient elution program as per the table below:

Retinol Elution Program Table

| Time (min) | Phase A (%) | Phase B (%) |
|---|---|---|
| 0 | 95 | 5 |
| 25 | 95 | 5 |
| Retention time of retinol t=9.2 min approximately | | |

6.6.2 Chromatographic Conditions for Retinyl-*trans*-4-tert-Butylcyclohexanol

Mobile Phase: A Phase Acetonitrile; B Phase 0.1% Formic Acid-Water
Column Temperature: 35°C
Flow Rate: 1.5 mL/min
Wavelength: 355 nm
Injection Volume: 10 μL Isocratic Elution Program as per the following table:)

Retinyl-*trans*-4-tert-Butylcyclohexanol Elution Program Table

| Time (min) | Phase A (%) | Phase B (%) |
|---|---|---|
| 0 | 100 | 0 |
| 25 | 100 | 0 |
| Retention time of Retinyl-*trans*-4-tert-Butylcyclohexanol t=17.0 min approximately | | |

6.7 Testing

Operate according to the procedures for high-performance liquid chromatography (HPLC) usage and maintenance:

I) Run the blank solvent, inject 2 times, record the chromatogram for up to 30 minutes;
II) Run at least 3 injections of the control solution 1, record the chromatogram for up to 30 minutes;
III) Run the control solution 2, inject 2 times, record the chromatogram for up to 30 minutes;
IV) Inject 2 samples of the test solution, record the chromatogram for up to 30 minutes;
V) Adjust the chromatogram scale, integrate, and print.

6.8 System Suitability Test

The RSD (relative standard deviation) of the main peak correction factor of the control solution should not exceed 3.0%;
The theoretical plate number in the chromatogram of the control solution, calculated based on retinol, should not be less than 3000, and the tailing factor should not exceed 2.0.)

6.9 Content Calculation

$$Cr=\frac{Mr\times p}{Vr}\;;$$

$$F=\frac{Cr}{Ar}\;;$$

$$\text{Content (\%)}=\frac{As\times F\times Vs}{Ms\times(1\text{-}Ws)}\times 100\%$$

The formula is as follows:

Cr represents the concentration of the reference solution (mg/mL);
Mr represents the weighed amount of the reference material (mg);
P represents the assigned content of the reference material; in this case, as provided by Coachchem Laboratory, P = 99.0%;
Vr represents the dilution factor of the reference solution;
F represents the correction factor for retinol;
Ar represents the peak area of the reference solution;
As represents the peak area of the sample solution;
Vs represents the dilution factor of the sample solution;
Ms represents the weighed amount of the sample material (mg);
Ws represents the moisture content of the sample material. If there is no moisture, then Ws = 0.00%.

6.10 The stability test results

The stability test results indicate that, except for the condition at 4°C, the stability of Retinol *trans*-4-t-Butylcyclohexanol ester is significantly higher than that of retinol under other conditions of light exposure, room temperature, 45°C, and 50°C. After being placed for 28 days under conditions of 4°C and room temperature, its content remains above 98%, showing minimal degradation and demonstrating good stability. The results are shown in Figures 7 to 11.
The above descriptions represent specific embodiments of the present invention. However, the scope of protection of the present invention is not limited thereto. Any modifications or substitutions readily conceived by those skilled in the art within the technical scope disclosed in the present invention shall be encompassed within the protection scope of the present invention. Therefore, the protection scope of the present invention shall be determined by the scope of protection as set forth in the claims.

## Claims

1. A type of small-molecule compound **characterized by** the following structural formula:

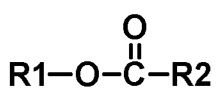
;

Wherein, R1 is selected from compounds represented by the following formula:

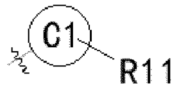
,

C1 represents a five-membered or six-membered carbon ring or heterocycle, while R11 represents alkyl or ester groups with multiple substitutions on the ring;

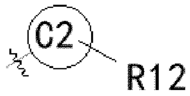

,

C2 is an aromatic ring, while R12 represents alkyl, ester, carbonyl, ether groups, or cyclic substituents formed by two adjacent substituents on the ring with multiple substitutions.

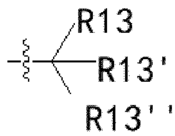

,

R13, R13', R13", whether identical or different, are chosen from hydrogen, ester groups, alkyl, alkenyl, and cycloalkenyl.
R2 is chosen from

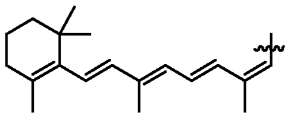 or 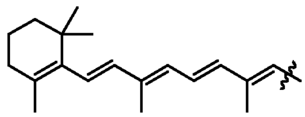.

2. A small molecular compound as described in claim 1, **characterized by**:
R1 is chosen from

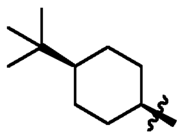

or

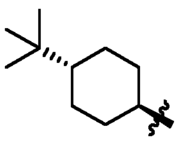 or 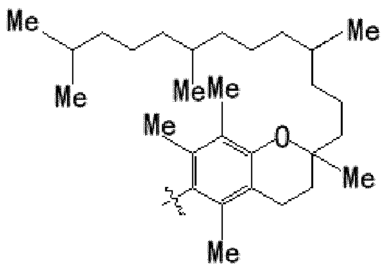 or 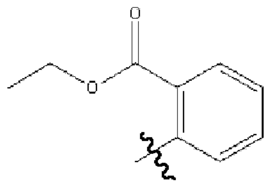 or 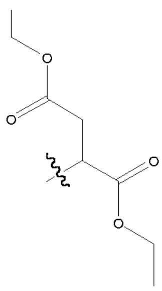

or

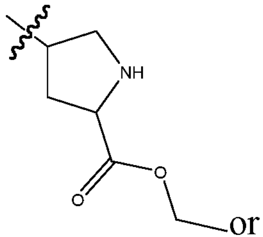 or 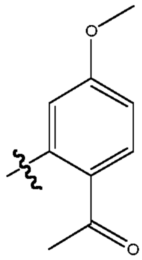 or 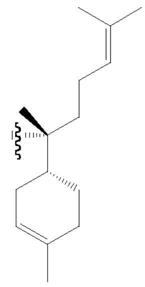 .

3. A small molecular compound as described in claim 1, **characterized by**:
The small molecular compound is selected from compounds represented by the following structures:

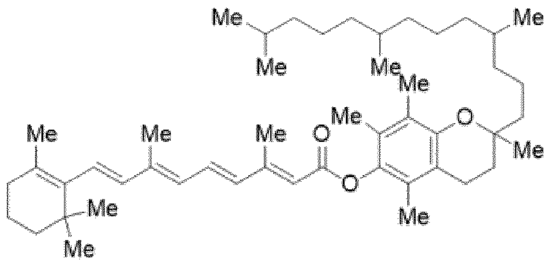

compound 1;

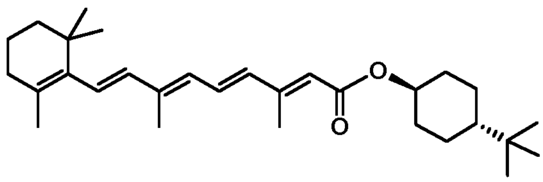

compound 2;

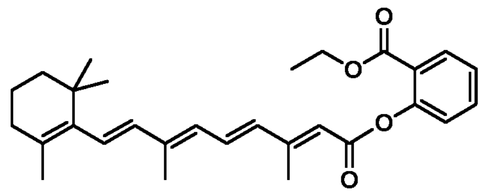

compound 3;

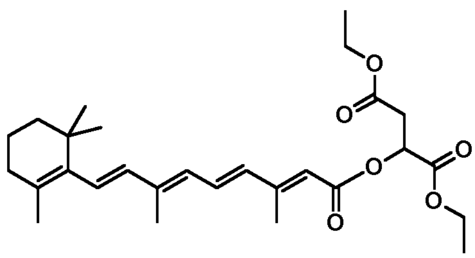

compound 4;

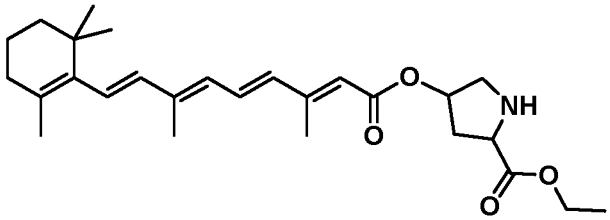

compound 5;

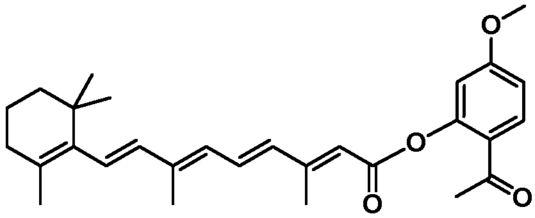

compound 6;

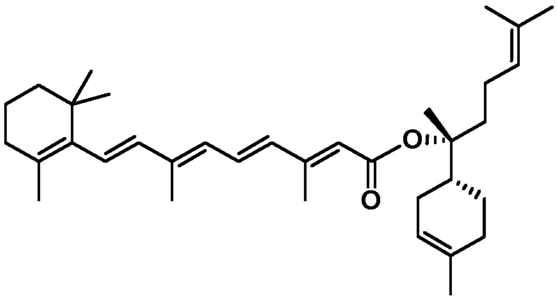

compound 7;

4. A small molecular compound as described in claim 1, **characterized by**:
The mentioned small molecular compound serves as an active ingredient in cosmetics.

5. A small molecular compound as described in claim 1, **characterized by**:
Containing at least one of the following uses:

C. Serving as an antioxidant.
D. Serving as an antioxidant for use in cosmetics.

6. A small molecular compound as described in claim 1, **characterized by**:

A. Serving as a DPPH radical scavenger.
B. Serving as a DPPH radical scavenger for use in cosmetics.

7. A small molecular compound as described in claim 1, **characterized by**:

C. Serving as an inhibitor of intracellular reactive oxygen species (ROS).
D. Serving as an inhibitor of intracellular reactive oxygen species (ROS) for use in cosmetics.

8. A small molecular compound as described in claim 1, **characterized by**:

Using a selenium-containing catalyst, under its catalytic action, carboxylic acids containing active unit A and

alcohols containing active unit B react to form small molecular compounds containing both active units A and B simultaneously.
Wherein, the carboxylic acid containing active unit A mentioned above has the general formula R2COOH;
The alcohol containing active unit B mentioned above has the general formula R1OH.

**9.** A small molecular compound as described in claim 8, **characterized by**:
The structural formula of the selenium-containing catalyst is as follows:

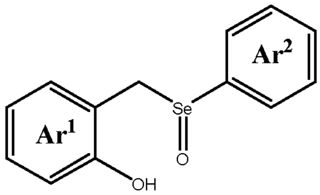
;

Wherein, the

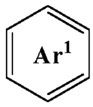
and
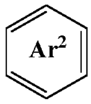

are benzene ring that may or may not contain substituents; these substituents are chosen from alkyl, alkoxy, fluoroalkyl, halogens, cyano, and amino groups.

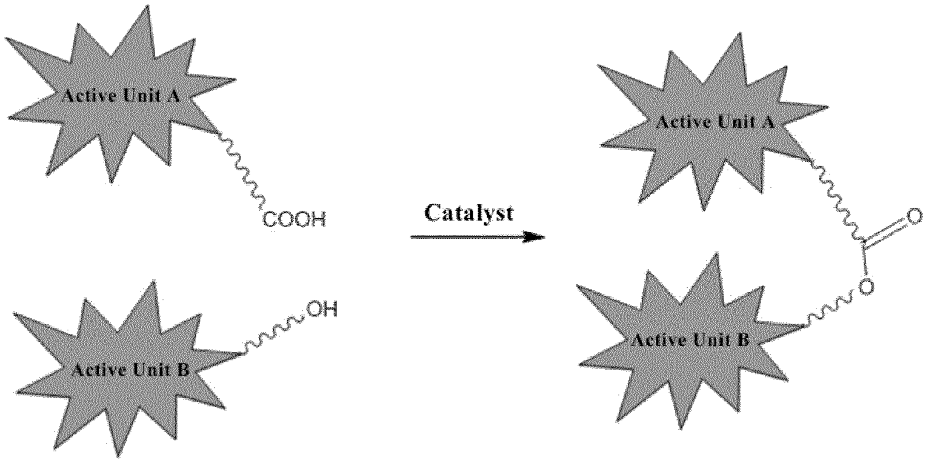


Figure 1-1

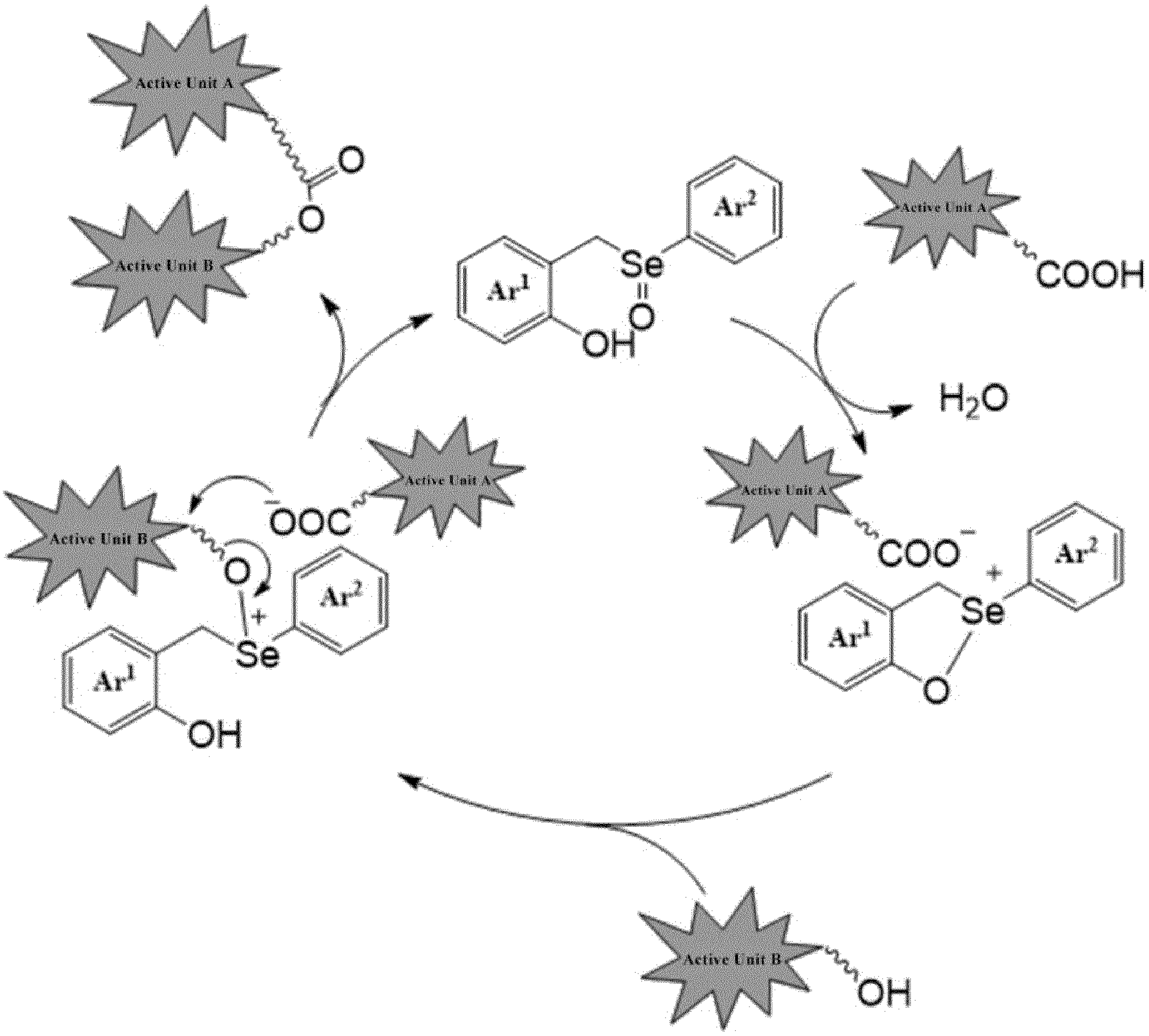

Figure 1-2

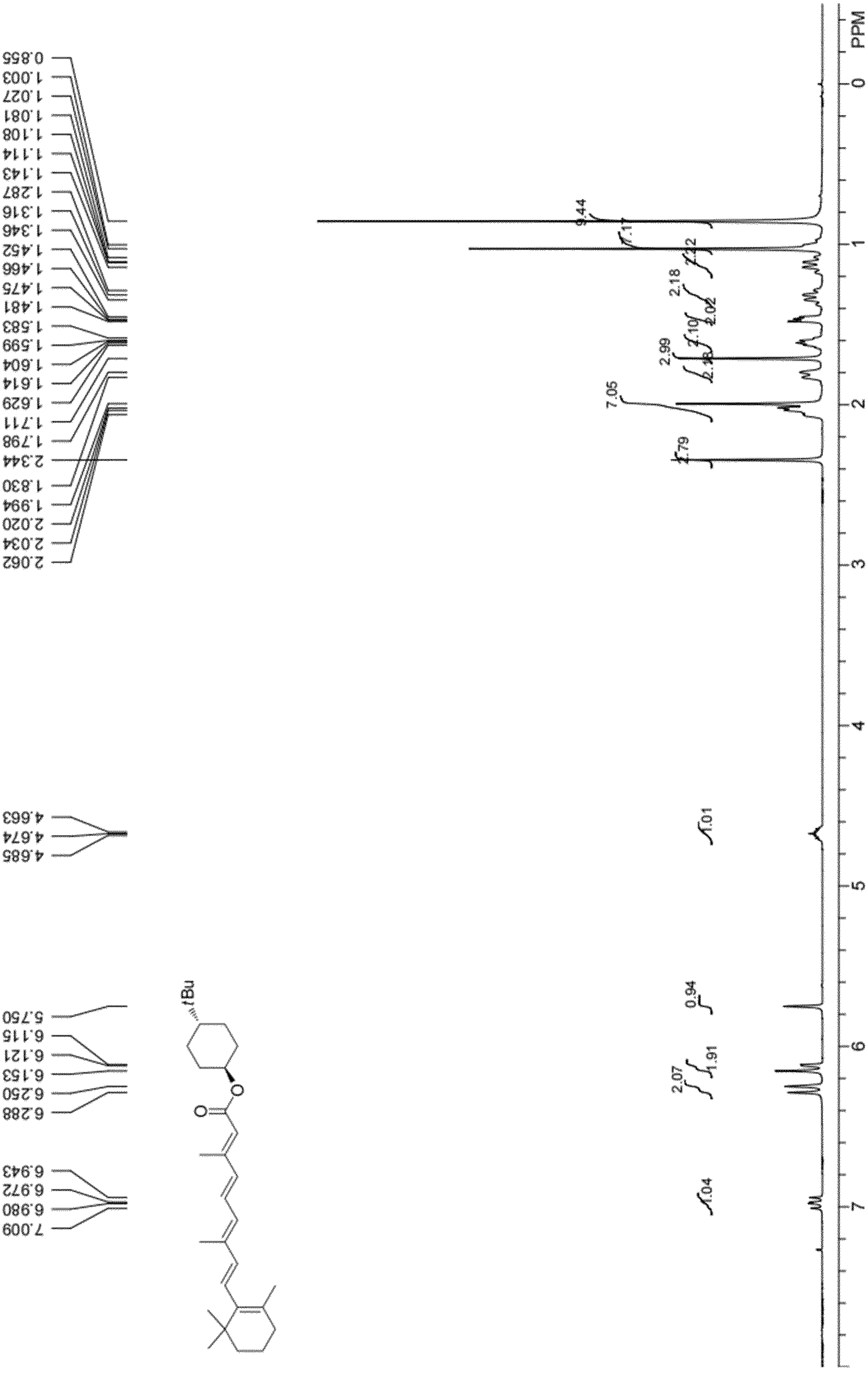
0.855
1.003
1.027
1.081
1.108
1.114
1.143
1.287
1.316
1.346
1.452
1.466
1.475
1.481
1.583
1.599
1.604
1.614
1.629
1.711
1.798
2.344
1.830
1.994
2.020
2.034
2.062
4.663
4.674
4.685
5.750
6.115
6.121
6.153
6.250
6.288
6.943
6.972
6.980
7.009
tBu
O
PPM
0
1
2
3
4
5
6
7

Figure 2-1

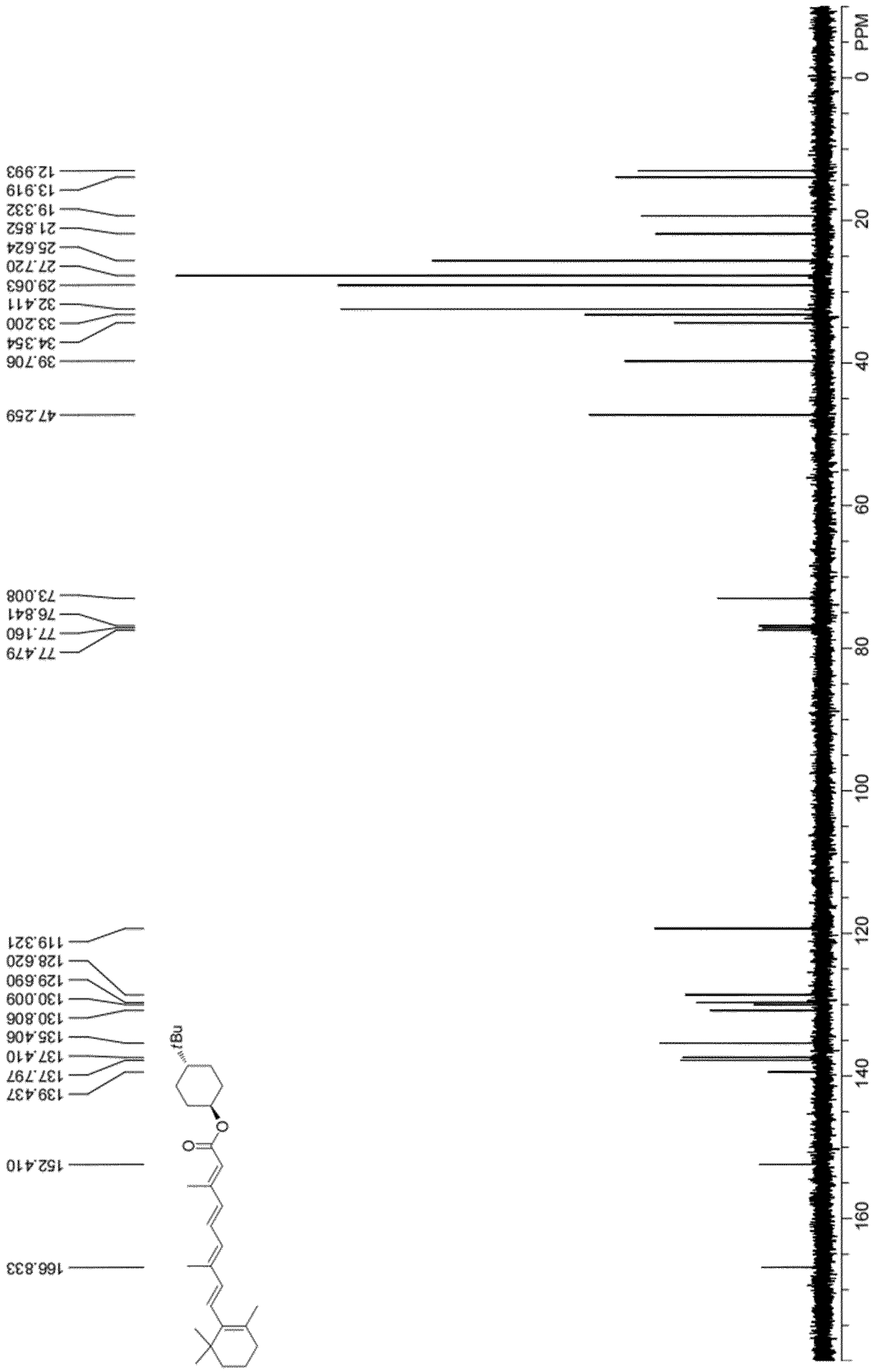
12.993
13.919
19.332
21.852
25.624
27.720
29.063
32.411
33.200
34.354
39.706
47.259
73.008
76.841
77.160
77.479
119.321
128.620
129.690
130.009
130.806
135.406
137.410
137.797
139.437
152.410
166.833
PPM
0
20
40
60
80
100
120
140
160

Figure 2-2

[redacted] Analytical Testing Center
Elemental Analysis Report

| | | | |
|---|---|---|---|
| Sample Number: | S221122-2009011-001 [redacted] | Status: | User has confirmed |
| Operator: | [redacted] | Contact information: | [redacted] |
| Sample Name: | KQ-1 | Estimated cost: | [redacted] |

| | | | |
|---|---|---|---|
| Analysis Project Requirements: | C,H | Elements Present in the Sample: | C,H,O |
| Solid, Melting Point: | | Liquid, Boiling Point: | |
| Sample Properties: | ☑ Regular ☐ Photosensitive ☐ Water-absorbent ☐ Volatile ☐ Toxicity ☐ Radioactive ☐ Explosion | | |
| Possible Molecular Formula or Structural Formula | C30H46O2 | Estimated Percentage Content: | C, 82.14; H, 10.57; O, 7.29 |
| Test Requirements: | ☑ Regular ☐ Anhydrous ☐ Sample Delivery by Phone ☐ Anhydrous Phone Sample Delivery | | |
| | ☐ Other | | |

## Test results

| | | | |
|---|---|---|---|
| Billing method: | Customization | Testing cost: | [redacted] |
| Remark: | Tester [redacted] | | |

| | | | | |
|---|---|---|---|---|
| Sample weight: | | 2.273 mg | 2.378 mg | 0.000 mg |
| Percent composition | C | 82.00 % | 82.03 % | 0.0000 % |
| | H | 10.67 % | 10.70 % | 0.0000 % |
| | | 0.0000 % | 0.0000 % | 0.0000 % |
| Remark | | | | |

| File name | Type | Version information | Operation |
|---|---|---|---|

Figure 2-3

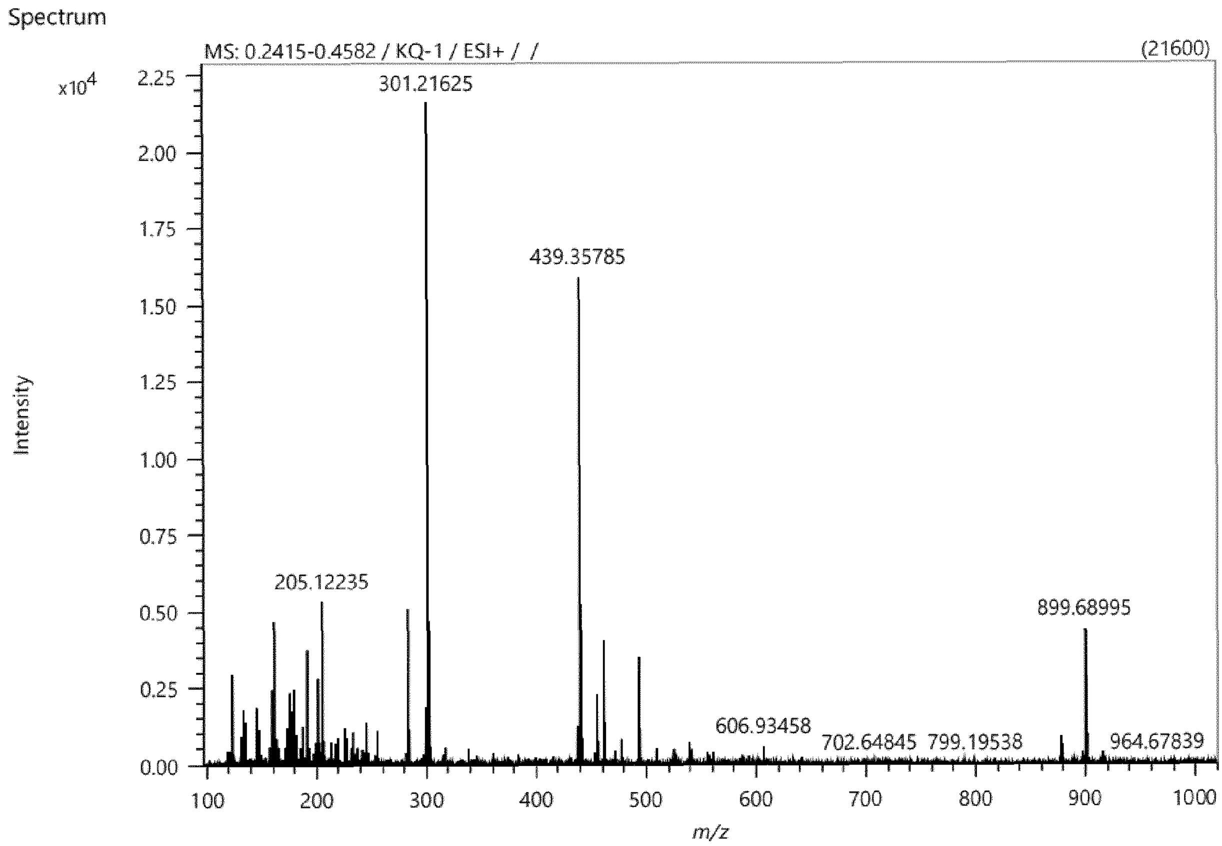


Figure 2-4

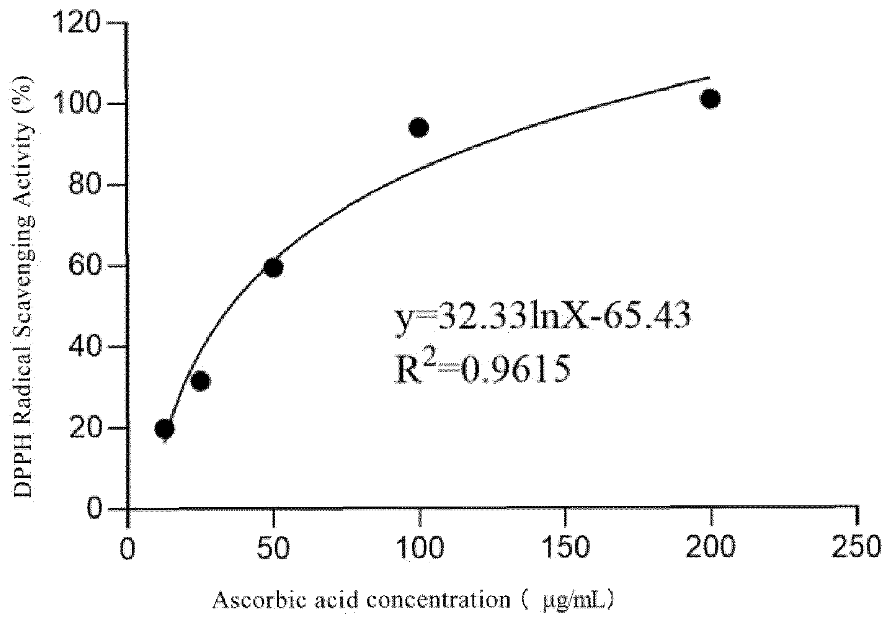


Figure 3

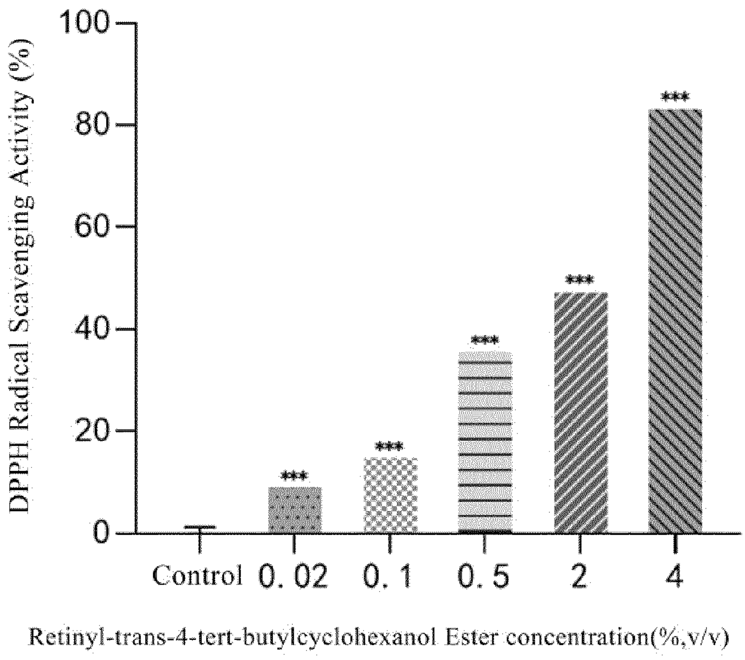


Figure 4

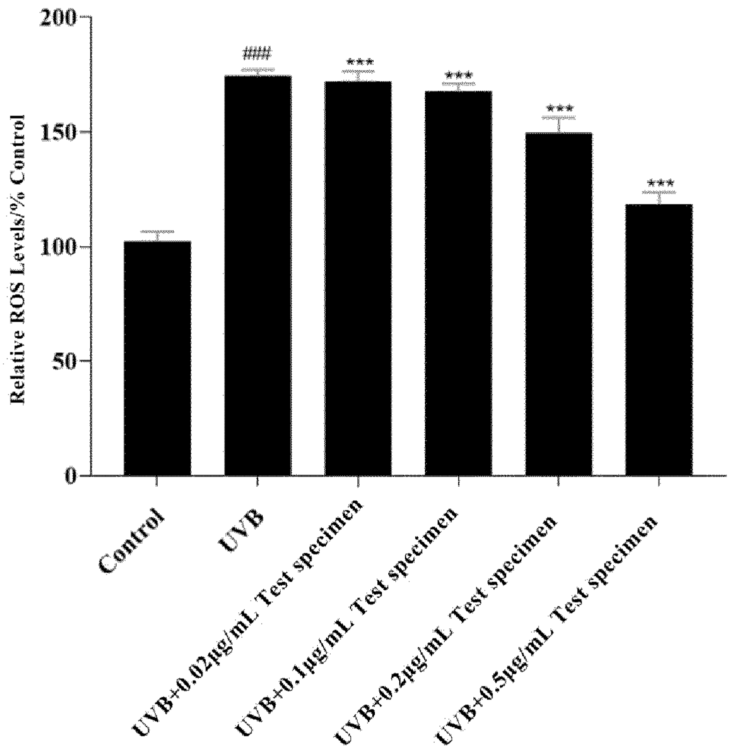


Figure 5

Skin Reactions in 30 Participants at Different Observation Times

| Number | Name (Initial) | Gender | Age | Negative Control | | | Test Substance | | |
|---|---|---|---|---|---|---|---|---|---|
| | | | | 0.5h | 24h | 48h | 0.5h | 24h | 48h |
| 1 | WCY | Female | 55 | 0 | 0 | 0 | 0 | 0 | 0 |
| 2 | DQQ | Male | 55 | 0 | 0 | 0 | 0 | 0 | 0 |
| 3 | WBJ | Female | 45 | 0 | 0 | 0 | 0 | 0 | 0 |
| 4 | TYY | Male | 45 | 0 | 0 | 0 | 0 | 0 | 0 |
| 5 | GPH | Female | 50 | 0 | 0 | 0 | 0 | 0 | 0 |
| 7 | SSJ | Male | 35 | 0 | 0 | 0 | 0 | 0 | 0 |
| 8 | SQ | Male | 32 | 0 | 0 | 0 | 0 | 0 | 0 |
| 9 | JHZ | Female | 35 | 0 | 0 | 0 | 0 | 0 | 0 |
| 10 | XS | Male | 52 | 0 | 0 | 0 | 0 | 0 | 0 |
| 11 | LL | Male | 43 | 0 | 0 | 0 | 0 | 0 | 0 |
| 12 | CJ | Female | 50 | 0 | 0 | 0 | 0 | 0 | 0 |
| 13 | DQL | Male | 22 | 0 | 0 | 0 | 0 | 0 | 0 |
| 14 | ZXF | Female | 31 | 0 | 0 | 0 | 0 | 0 | 0 |
| 15 | ZCY | Male | 38 | 0 | 0 | 0 | 0 | 0 | 0 |
| 16 | ZMJ | Female | 31 | 0 | 0 | 0 | 0 | 0 | 0 |
| 17 | CY | Male | 31 | 0 | 0 | 0 | 0 | 0 | 0 |
| 18 | MZ | Male | 43 | 0 | 0 | 0 | 0 | 0 | 0 |
| 19 | WXT | Female | 34 | 0 | 0 | 0 | 0 | 0 | 0 |
| 20 | LRY | Male | 24 | 0 | 0 | 0 | 0 | 0 | 0 |
| 21 | JYP | Male | 48 | 0 | 0 | 0 | 0 | 0 | 0 |
| 22 | GHQ | Female | 55 | 0 | 0 | 0 | 0 | 0 | 0 |
| 23 | LY | Male | 43 | 0 | 0 | 0 | 0 | 0 | 0 |
| 24 | GXY | Female | 55 | 0 | 0 | 0 | 0 | 0 | 0 |
| 25 | WLH | Female | 34 | 0 | 0 | 0 | 0 | 0 | 0 |
| 26 | YLN | Male | 45 | 0 | 0 | 0 | 0 | 0 | 0 |
| 27 | XP | Female | 45 | 0 | 0 | 0 | 0 | 0 | 0 |
| 28 | HCR | Female | 45 | 0 | 0 | 0 | 0 | 0 | 0 |
| 29 | BYJ | Male | 42 | 0 | 0 | 0 | 0 | 0 | 0 |
| 30 | HFF | Female | 29 | 0 | 0 | 0 | 0 | 0 | 0 |
| 31 | LNN | Female | 30 | 0 | 0 | 0 | 0 | 0 | 0 |

Note: The results are based on a systematic sampling method, including data from the first 30 eligible participants.

Figure 6

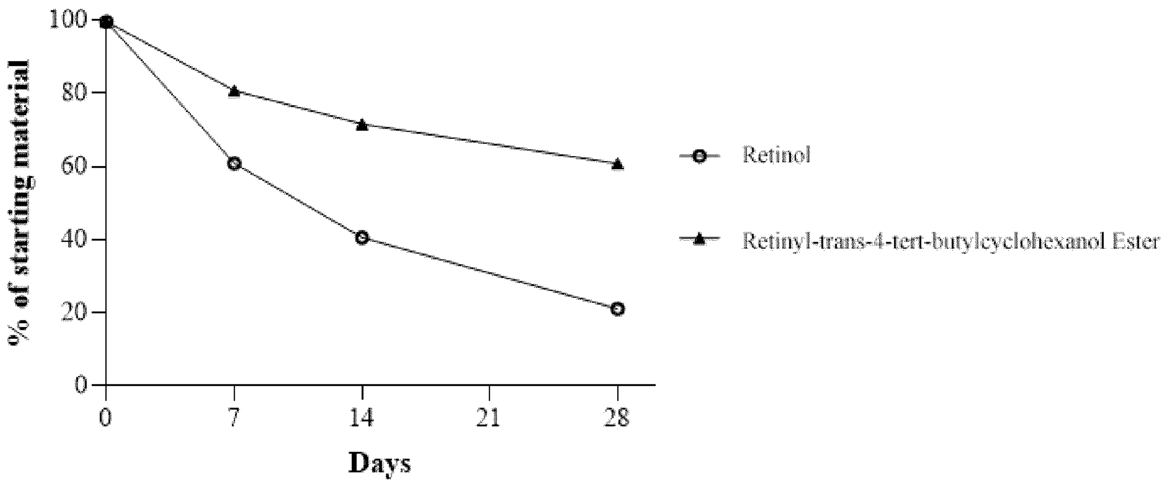


Figure 7

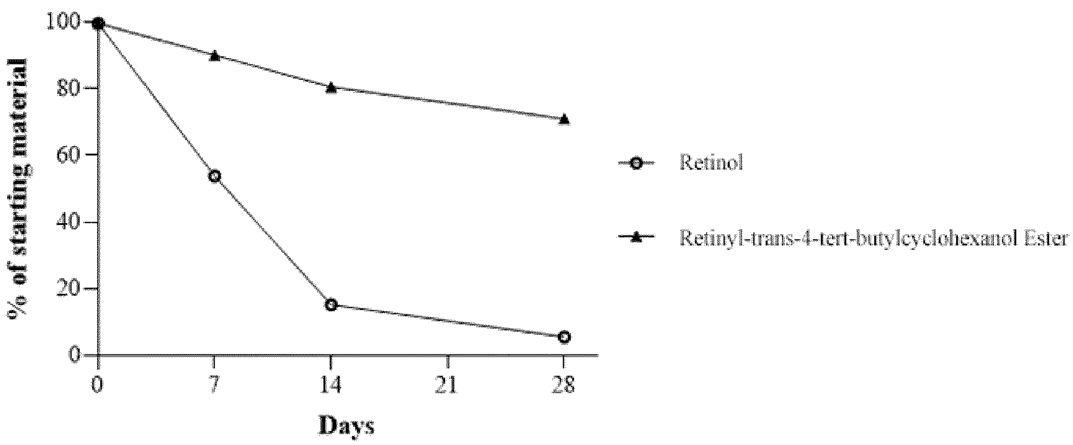


Figure 8

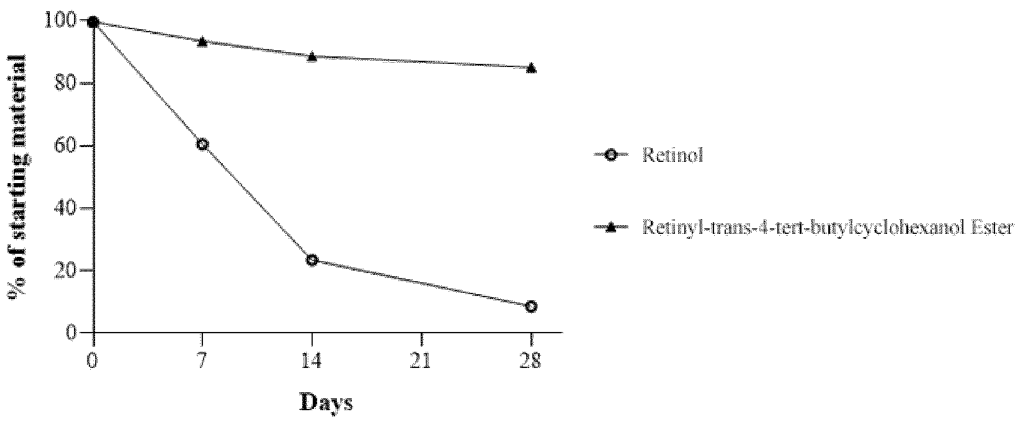


Figure 9

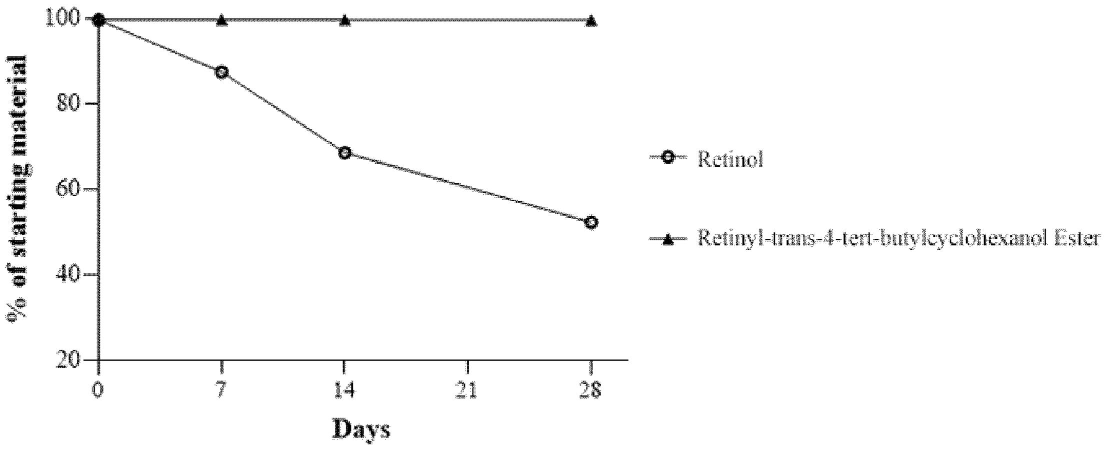


Figure 10

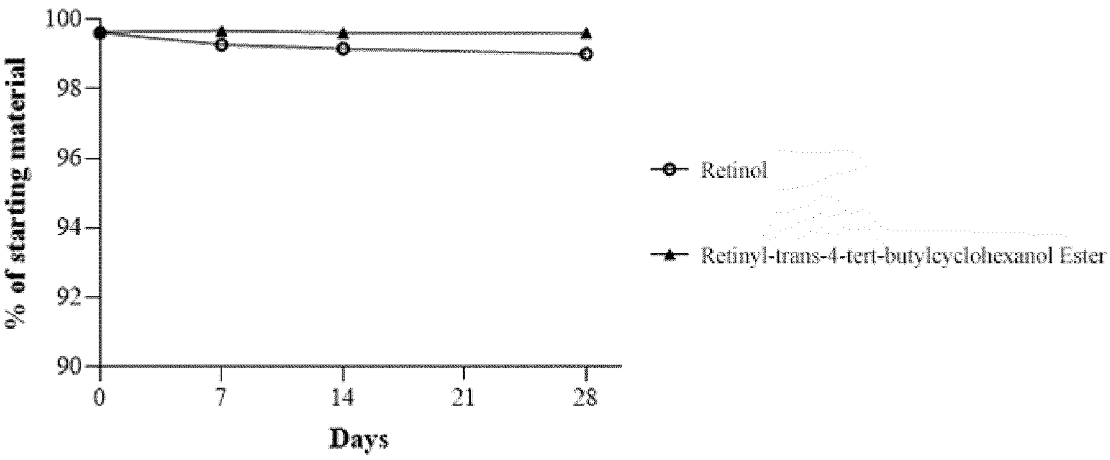


Figure 11

**INTERNATIONAL SEARCH REPORT**

International application No. **PCT/CN2023/093505**

**A. CLASSIFICATION OF SUBJECT MATTER**

C07D311/72(2006.01)i; C07C403/20(2006.01)i; C07D207/16(2006.01)i; A61P39/06(2006.01)i; C07C391/02(2006.01)i; B01J31/02(2006.01)i; A61K8/67(2006.01)i; A61Q19/08(2006.01)i

According to International Patent Classification (IPC) or to both national classification and IPC

**B. FIELDS SEARCHED**

Minimum documentation searched (classification system followed by classification symbols)

IPC:C07D,C07C,A61P,A61Q,B01J,A61K

Documentation searched other than minimum documentation to the extent that such documents are included in the fields searched

Electronic data base consulted during the international search (name of data base and, where practicable, search terms used)

VEN, CNKI, CNTXT, 万方 WANFANG, REGISTRY, CAPLUS, CASREACT: 上海克琴科技, 视黄醇, 硒催化剂, 视黄酸, 维生素A酸, 生育酚, 维生素e, 活性氧, 自由基清除, tocopheryl retinoatr, tocoperyretinoate, tocoretinate, dpph, ros, free radical, reactive oxygen , STN structure search, reaction searc

**C. DOCUMENTS CONSIDERED TO BE RELEVANT**

| Category* | Citation of document, with indication, where appropriate, of the relevant passages | Relevant to claim No. |
|---|---|---|
| X | CN 106631950 A (SUZHOU PHARMPOWER MEDICINE TECHNOLOGY CO., LTD.) 10 May 2017 (2017-05-10)<br>see claim 1, and description, page 3 paragraph 37 | 1-7 |
| PX | CN 115925666 A (SHANGHAI COACHCHEM TECHNOLOGY CO., LTD.) 07 April 2023 (2023-04-07)<br>see claims 1-9 | 1-9 |
| X | CN 114907304 A (SHANGHAI COACHCHEM TECHNOLOGY CO., LTD.) 16 August 2022 (2022-08-16)<br>see description, page 1 paragraphs 3-4 | 1-7 |
| A | JEON, H. S. et al. "Stereoselective and Convergent Syntheses of Retinoic Acid and its Ester Derivatives by the Sulfone Olefination Reaction"<br>*Synthesis*, Vol. 17, 15 October 2004 (2004-10-15),<br>pp. 2813-2820 | 1-9 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 September 2023** | **06 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2023/093505

C. DOCUMENTS CONSIDERED TO BE RELEVANT

| Category* | Citation of document, with indication, where appropriate, of the relevant passages | Relevant to claim No. |
|---|---|---|
| A | 俞磊 (YU, Lei). "硒催化绿色合成反应进展 (Progresses in Se-catalyzed Green Synthetic Reactions)" *化学试剂 (Chemical Reagents),* Vol. 41, No. 6, 29 December 2018 (2018-12-29), pp. 545-549 | 1-9 |
| A | 左玉等 (ZUO, Yu et al.). "维生素类化合的研究进展 (The Direction of Research and Development of Vitamins)" *粮食与油脂 (Cereals & Oils),* Vol. 28, No. 9, 30 September 2015 (2015-09-30), pp. 1-5 | 1-9 |

Form PCT/ISA/210 (second sheet) (July 2022)

**INTERNATIONAL SEARCH REPORT**
**Information on patent family members**

International application No.
**PCT/CN2023/093505**

| Patent document cited in search report | Publication date (day/month/year) | Patent family member(s) | Publication date (day/month/year) |
|---|---|---|---|
| CN 106631950 A | 10 May 2017 | None | |
| CN 115925666 A | 07 April 2023 | CN 115925666 B | 14 July 2023 |
| CN 114907304 A | 16 August 2022 | None | |

Form PCT/ISA/210 (patent family annex) (July 2022)